# EUROPEAN PATENT SPECIFICATION

(11) **EP 1 423 101 B1**
(45) Date of publication and mention of the grant of the patent: **02.03.2011**
(21) Application number: 02776988.4
(22) Date of filing: 04.09.2002
(51) Int. Cl.: A61K 9/70, A61P 31/10

(54) **USE OF A PLASTER FOR THE TREATMENT OF ONYCHOMYCOSES**
VERWENDUNG EINES PFLASTERS ZUR BEHANDLUNG VON NAGELPILZERKRANKUNGEN
UTILISATION DE PANSEMENT ADHESIF POUR LE TRAITEMENT D'ONYCHOMYCOSES

(30) Priority: 04.09.2001 EP 01121201; 12.09.2001 US 318354 P
(43) Date of publication of application: 02.06.2004
(73) Proprietor: Trommsdorff GmbH & Co.KG Arzneimittel, 52475 Alsdorf (DE)
(72) Inventor: SUSILO, Rudy, 50999 Köln (DE)
(74) Representative: Arth, Hans-Lothar
(86) International application number: PCT/EP2002/009911
(87) International publication number: WO 2003/020249

(56) References cited:
- WO-A-94/08591
- WO-A-99/40955
- US-A- 5 181 914
- US-A- 5 464 610
- US-A- 5 696 164
- US-A- 5 753 256
- US-A- 5 861 142
- US-A- 5 993 790
- US-B1- 6 303 140

## Description

The present invention relates to the use of a layer for the manufacture of plasters without any antifungal agent for prophylaxis and/or treatment of onychomycosis or dysfunctions or disorders of nails, and methods for prophylaxis and/or treatment of onychomycosis or dysfunctions or disorders of nails in combination with systemic antimycotics using said plasters.

### Background of the invention

Dysfunction and disorder of nails caused by e.g. onychomycosis is an increasingly common and recalcitrant fungal nail infection world-wide.

A dysfunction or disorder of nails is often induced or caused by fungal infections of the nails and/or nail beds. Particularly in the later stages of such an infection said dysfunctions or disorders are difficult to treat. Said dysfunctions or disorders of nails comprise, for example onychomycosis, onychocryptosis, and onychodystrophy. Bacteria like staphylococci or yeast may cause the bacterial infection paronychia, a superficial infection of the nail wall.

The current treatment of onychomycosis generally falls into three categories:
a) systemic administration of antifungals,
b) surgical removal of all or part of the nail followed by topical treatment of the exposed tissue, or
c) topical application of conventional creams, lotions, gels or solutions on the infected nail, frequently including the use of bandages to keep these dosage forms in place on the nails.

Systemic, generally oral administration of an antifungal agent for the treatment of onychomycosis requires a long term treatment (6 months and longer) and the administration of high doses (200 - 400 mg per day) of an antifungal agent. Surgical removal of the whole nail or parts thereof is painful, requires bandaging of the whole toe or finger and causes undesirable cosmetic appearance. Topical dosage forms such as gels, creams, solutions, lotions, lacquers etc. have the drawback that the pharmaceutically active agent is not in sufficient intimate contact with the nail.

Plasters for the treatment of onychomycosis are known. For instance, WO-A-99/40955 discloses a pressure sensitive adhesive matrix patch for the treatment of onychomycosis. This device for treating fungal infections of toenails and fingernails is made up of an occlusive backing layer and a pressure-sensitive adhesive matrix layer wherein an effective amount of an antifungal agent is uniformly dispersed, optionally with a chemical enhancer. The matrix layer has a first surface adhering to the backing layer and a second surface adapted to be in diffusional contact with the infected nail and surrounding skin area.

A method for treating onychomycosis is described in US-A-5 464 610. Within said method a plaster preparation is used comprising salicylic acid or a salt, ester or mixture thereof. Said plaster preparation is attached to a carries and the salicylic acid is present in the plaster preparation in an amount ranging from 10 to 80% by weight of the preparation.

Nail evulsion compositions and methods for evulsing nails and treating nail and nail bed infections are disclosed in US-A-5 993 790. Claimed is a topical nail enamel composition comprising water-based nail lacquer, a preservative, urea, and a natural additive. Said nail enamel composition is suitable for the treatment of fungal, yeast, and bacterial infections of the nails and the nail beds.

US-A-5 753 256 discloses a plaster for the treatment of nail mycoses which consists of a flexible covering film, a layer of an acrylate polymer matrix, inseparably linked to said covering film, and comprises an active compound selected form miconazole, econazole, isoconazole, tioconazole, terconazole, oxiconazole, ketoconazole, itraconazole, tolciclate, sulbentine, haloprogin, griseofulvin, cyclopirox, terbinafin, and salts of these compounds.

US-A-5 181 914 discloses an adhesive gel pad for treating onychomycosis comprising an occlusive layer, a permeation enhancer, and an antimycotic agent.

US-A-6 303 140 discloses a plaster for treating warts, corns and calluses that contains as an active ingredient salicylic acid. US-A-6 303 140 also discloses when the plaster is used to treat onychomycosis antifungal agents such as clotrimazole, butenafine, terbinafine or miconazole can be added.

All the plasters of the state of the art for preventing and/or treating of nail disorders have in common that at least one antifungal agent is contained in that plaster.

It is object of the present invention to provide a layer for use in the manufacture of a plaster without any antifungal agent for prophylaxis and/or treatment of onychomycosis and of other dysfunctions or disorders of nails.

This object is solved by the use of the plasters of the independent claims and the use of said plaster. Further advantageous features, aspects and details of the invention are evident from the dependent claims, the description, and the examples of the present application.

### Description of the invention

The present application relates to plasters for prophylaxis and/or treatment of a dysfunction or disorder of nails. Said plasters comprise a layer being designed to be in close contact with the nail and optionally with the surrounding skin. Said layer consists of:
a) an adhesive;
b) at least one skin and/or nail permeation enhancer; and
c) at least one additive selected from the group comprising binders, cross linkers, softeners, solvents, fillers, and/or antioxidants.

Plasters constructed of two or more layers are also useful for the purposes disclosed herein. A preferred embodiment comprises a layer which is separated into a backing layer and a contact layer wherein the contact layer is attached to said backing layer and is designed to be in close contact with the nail and optionally with the surrounding skin. Said contact layer consists of:
a) an adhesive;
b) at least one skin and/or nail permeation enhancer; and
c) at least one additive selected from the group comprising binders, cross linkers, softeners, solvents, fillers, and/or antioxidants.

The flexible backing layer holds and presses the plaster against the nail and skin in order to increase migration from the contact layer into the nail, nail beds, and surrounding skin. Furthermore, the backing layer protects the contact layer form being contaminated. A preferred embodiment of said plaster comprises a colorless backing layer or a backing layer having an amber-like color. Another preferred embodiment comprises a flexible and/or occlusive backing layer. The plaster has sufficient flexibility in order to seal the affected nail exactly even if the nail has an uneven surface.

Surprisingly, it was found that the inventive plaster containing no antifungal agent is very effective in order to treat dysfunctions or disorders of nails.

The backing layer is preferably made of polyethylene (such as LDPE, Plastotrans^{®}), polypropylene, polyurethane, polyester (such as Revatrans^{®}, TRICON GmbH, Freiburg), Guttagena^{®} PVC NBR foil (such as Guttagena^{®} WK 68, Kalle Pentaplast, Germany), cotton, cotton/viscose, silk, polyethylenterephthalate (such as Hostaphan^{®} RN 36 sil; Hostaphan^{®} RN 100 sil, Loparex, Apeldoorn, The Netherlands), ethylene-methacrylic acid coplymers and/or mixtures of these materials. More preferably are siliconized polymers and/or copolymers.

As used herein, the term "contact layer" refers to a biocompatible adhesive which is preferably a gel-like or rubber-like adhesive containing at least one skin and/or nail permeation enhancer and optionally further binders and/or additives with special biological functions suitable to allow and support migration and penetration of a compound into the nails, nail beds, and the associated skin. The contact layer is inseparably linked with the backing layer, preferably with a flexible and/or occlusive backing layer.

The inventive plaster can be manufactured in any suitable shape, such as round, oval, rectangular or quadratic shape. Preferred plaster sizes are 0.5 cm², 0.85 cm², 1.5 cm², 2.3 cm², 2.5 cm², and 4.0 cm².

The dysfunction or disorder of nails comprises onychomycosis, onychocryptosis, nail psoriasis, melanonychia striata, white line disease, eczema, chronic onychia, discolored nails, thickened nails, and onychodystrophy. Said dysfunctions or disorders of nails are most likely caused or induced by fungi, yeasts, and/or bacteria. It is known that dermatophytes and yeasts are responsible for the majority of onychomycosis cases.

Onychomycosis, as a fungal infection, is regarded as a subgroup of onychodystrophy. Onychodystrophy comprises a number of nail dysfunctions and disorders such as onychocryptosis, melanonychia striata, white line disease, chronic paronychia, discolored nails, thickened nails, Unguis inflexus, coilonychia, scleronychia, onychogryphosis, onychauxis, onychoschisis, onychorrhexis, trachyonychia, cleaved and split nails.

The most prominent group of onychodystrophy apart from onychomycosis are induced by diseases of the skin such as neurodermitis (atopic eczema), and psoriasis. Furthermore, bacterial or viral infections are capable of causing or inducing onychodystrophy.

Also drugs such as antibiotics, anticoagulative agents, ACE inhibitors, betablockers, thiazides, cytostatic agents and the like are known to cause onychodystrophy. Another reason for onychodystrophy are systemic diseases such as avitaminoses, kidney failure, and heart failure. Another reason for onychodystrophy is the contact with chemical compounds such as acids, bases, oxidants and the like which cause burns, cauterizations, and also physical influences resulting in mechanical destruction of the nail plate. Finally, idiopathic causes exist for dysfunctions and/or disorders of the nail.

As used herein, the term "nail" refers to fingernails and toenails of mammals, especially humans.

A preferred embodiment of said plaster comprises a contact layer designed in that way that said contact layer seals the infected nail almost perfectly which results in an almost quantitative exclusion of air. In case of fungal nail infections caused by aerobic fungi the exclusion of air, that means more precisely the exclusion of atmospheric oxygen, increases the effectiveness of the inventive plaster. Depriving aerobic fungi of atmospheric oxygen can be achieved by forming an oxygen barrier over the exposed surface of the infected nail and the surrounding tissue. The oxygen barrier is formed by the contact layer which seals the infected nail and the surrounding tissue almost perfectly. Furthermore, the inventive plaster may optionally contain an additional oxygen scavenger. Suitable oxygen scavenger comprise transition metal chelates or complexes with, for instance and/or polycarboxylic acids, or oxidizable organic acids or alcohols in combination with a catalyzing agent.

It could be proven that the inventive plaster shows high efficacy against fungi such as *Trichophyton mentagrophytes, Trichophyton rubrum, Trichophyton interdigitale, T. schönleinii, T. verrucosum, T. violaceum, T. tonsurans, Trichophyton spp., M. canis, Candida albicans, C. guillermondii, C. krusei, C. parapsilosis, C. tropicalis, C. glabrata, Candida spp., Microsporum spp., Microsporum canis, Microsporum audonii, Microsporum gypseum, M. ferrugineum, Trichosporum beigelii, Trichosporum inkiin, Aspergillus niger, Alternaria, Acremonium, Fusarium, and Scopulariopsis* in comparison with other pharmaceutically active antifungal agents like econazole, ketonazole, miconazole, or bifonazole.

Preferred is the use of the inventive plasters for the prophylaxis and treatment of nail infections caused and/or mediated by *Candida albicans.*

Suitable skin and/or nail permeation enhancer are well known to a person skilled in the art and may be selected from of fatty acids, fatty acid esters, fatty acid amides, fatty alcohols, 2-(2-ethoxyethoxy)-ethanol, esters of glycerol, glycerol monolaurate, propylene glycol, polyethylene glycols, unsaturated polyglycolized glycerides (Labrafil M1944CS^{®}, Gattefosse), saturated polyglycerides (Labrasol^{®}, Gattefosse), a partial glyceride of ricinoleic acid (Softigen^{®}, Hüls), Labrafac Hydro WL1219^{®} (Gattefosse), Estasan^{®} (Gattefosse), α-hydroxy acids, dimethylsulfoxide, decylmethylsulfoxide, pyrrolidones, lactic acid, myristol, isopropyl myristate, dimethylformamide, dimethylacetamide, sodium dodecylsulfate, phospholipides, Transcutol^{®} (Gattefosse), Eutanol^{®} (Henkel), as well as mixtures comprising oleic acid / 2-(2-ethoxyethoxy)-ethanol, oleic acid / Labrafil^{®}, and oleic acid / Labrafac^{®} (Gattefosse), preferably in a ratio of approximately 1:1, and the like. Also enzyme components, such as proteolytic enzymes which facilitate permeation of chemical substances through the hardened nail or keratin tissue, can be used as permeation enhancer.

Examples for most common fatty acids are capric-, lauric-, myristic-, palmitic-, margaric-, stearic-, arachidic-, behenic-, lignoceric-, myristoleic-, palmitoleic-, petroselinic-, oleic-, vaccenic-, gadoleic-, gondoic-, urucic-, nervonic-, linoleic-, γ-linolenic-, dihomo-γ-linolenic-, arachidonic-, 7,10,13,16-docosatetraenoic-, 4,7,10,13,16-docosapentaenoic-, α-linolenic-, stearidonic-, 8,11,14,17-eicosatetraenoic-, 5,8,11,14,17-eicosapentaenoic-, 7,10,13,16,19-docosapentaenoic-, 4,7,10,13,16,19-docosahexaenoic-, 5,8,11-eicosatrienoic-, tariric-, santalbic-, stearolic-, 6,9-octadecenynoic-, pyrulic-, crepenynic-, heisteric-, t8,t10-octadecadiene-12-ynoic-, 5,8,11,14-eicosatetraynoic-, cerebronic-, hydroxynervonic-, brassylic-, and thapsic acid. Also useful are the lower alkyl ester and amides of said fatty acids or the corresponding alcohols thereof. The glycerol esters may also contain one or more of said fatty acids.

The skin and/or nail permeation enhancer supports and increases the penetration and permeation of an agent through the skin and into the nails and nail beds. The term "penetration enhancement" or "permeation enhancement" relates to an increase in the permeability of a biological membrane or skin and nails. Skin and/or nail permeation enhancer are mostly used for increasing the rate at which an agent permeates through said membrane. The effect of permeation enhancement can be determined by the use of a diffusion cell apparatus as described by Merrit et al. (Diffusion Apparatus for Skin Penetration, J. Controlled Release, 1984, 1, 161-162) measuring the rate of diffusion of an agent through animal or human skin.

The inventive plaster preferably contains said skin and/or nail permeation enhancer in the contact layer in an amount of between 0.1% to 30% by weight of the adhesive, preferably 0.1% to 15% by weight of the adhesive, more preferably 0.5% to 10%, and most preferably 0.7% to 6% by weight of the contact layer.

The inventive plaster comprises at least one further additive selected from the group comprising binders, cross linkers, softeners, solvents, fillers, and/or antioxidants.

Said additive or said additives, if present, are contained in the layer in an amount of between 2% to 80% by weight of the contact layer, preferably 5% to 40% by weight of the contact layer, more preferably between 8% to 30%, even more preferably between 12% to 25%, and most preferably in an amount between 15% to 20% by weight of the contact layer.

Binders characterize substances that bind or "glue" powders together and make them cohesive by forming the adhesive layer, thus serving as a further "adhesive" in the formulation. Suitable binders include non-natural sugars, natural sugars such as sucrose, starches derived from wheat, corn rice and potato; synthetic and natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate, polyethylene glycol and waxes.

If present, the amount of binder in the adhesive can range from about 1 to 50% by weight of the adhesive, preferably from 10 to 50% by weight of the adhesive, more preferably from 20 to 50% by weight, even more preferably from 30 to 40% by weight.

Cross linkers may be selected from the group comprising cross linking agents such as aluminum acetylacetonate, acrylate-vinylacetate copolymer, aluminum acetonate, titanium acetylacetonate, titanium acetonate, and succinic acid.

If present, the amount of cross linkers in the adhesive can range from about 0.01 to 30% by weight of the adhesive, preferably from 0.1 to 50% by weight of the adhesive, more preferably from 10 to 50% by weight, even more preferably from 30 to 40% by weight.

Softener may be chosen from the group comprising dibutylsebacate (DBS), Macrogol^{®} (Clariant, Frankfurt, Germany) and the like.

If present, the amount of softener in the adhesive can range from about 0.001 to 25% by weight of the adhesive, preferably from 0.01 to 10% by weight of the adhesive, more preferably from 0.1 to 6% by weight, even more preferably from 0.5 to 3% by weight.

Suitable solvents for the inventive plaster may be selected form purified water; ketones such as acetone, butanone, 2-pentanone, 3-pentanone; alcohols such as ethanol, propanol, isopropanol, butanol, isobutanol, sec.-butanol, tert.-butanol; esters such as acetic acid ethyl ester, acetic acid propyl ester and the like. Furthermore, mixtures of said solvents can also be used. Suitable co-solvents may be used together with the above-mentioned solvents or mixtures of solvents, said co-solvents may be selected from the group comprising lactic acid, succinic acid, urea, Miglyol^{®} 812 (Chemische Werke Hüls, Marl, Germany), triglycerides, ethyloleate, glycerylmonododecanoate, olein, oleate, Macrogol^{®} 6000, and lecithin.

If present, the amount of solvents or the total amount of solvents and co-solvents in the adhesive can range from about 0.5 to 70% by weight of the adhesive, preferably from 3 to 60% by weight of the adhesive, more preferably from 10 to 50% by weight, even more preferably from 20 to 40% by weight, and most preferably from 10 to 30% by weight of the adhesive.

Fillers may be chosen from the group comprising silica, silicic acid, preferably colloidal silica and colloidal silicic acid, lactose, Aerosil^{®} such as Aerosil^{®} 200 (Degussa-Hüls, Frankfurt, Germany), starch, Bentonit^{®} (Südchemie, Mannheim, Germany) and the like.

If present, the amount of fillers in the adhesive can range from about 0.01 to 15% by weight of the adhesive, preferably from 0.1 to 10% by weight of the adhesive, more preferably from 0.3 to 6% by weight, even more preferably from 0.5 to 3% by weight.

Butylhydroxytoluene (BHT) may be mentioned as an example for a suitable antioxidant. Antioxidants are well known to a person skilled in the art and may be selected form the antioxidants of the state of the art.

If present, the amount of antioxidants in the adhesive can range from about 0.001 to 10% by weight of the adhesive, preferably from 0.005 to 6% by weight of the adhesive, more preferably from 0.01 to 3% by weight, even more preferably from 0.05 to 1% by weight.

Suitable adhesives for the inventive plaster may comprise acrylic copolymers, also known as "acrylic adhesives", like National Starch Durotak^{®} 80-1196, National Starch Durotak^{®} 387-2825, or Monsanto Gelva 737; polyacrylamide; rubber-based adhesives, also called "rubber adhesives", such as polyisobutylene (PIB) (e.g. Adhesive Research MA-24), polyisoprene, styrene-isoprene copolymers, or urethane rubbers; and silicone based adhesives, so called "silicone adhesives", such as Dow Bio-PSA.

The adhesives that may be used according to the invention represent a polymer, preferably an acrylate copolymer. Suitable monomers or mixtures of monomers for the manufacture of said acrylate polymer comprise methyl acrylate, methyl methacrylate, butyl acrylate, butyl methacrylate, isooctyl acrylate, isooctyl methacrylate, aminoalkyl acrylate, aminoalkyl methacrylate, aminoalkyl methacrylate copolymers (such as EUDRAGIT^{®} E 100, EUDRAGIT^{®} RL, EUDRAGIT^{®} RS, EUDRAGIT^{®} NE 30 D commercially available from Röhm, Degussa-Hüls Group), hydroxyethyl acrylate, hydroxyethyl methacrylate, 2-ethylhexyl acrylate, 2-ethylhexyl methacrylate, acrylic acid, methacrylic acid, vinyl acetale, and glycidyl methacrylate. Acrylate-based adhesives are commercially available from National Starch Chemical B. V., Zutphen, NL, under the name Durotak^{®}. Examples of said product class are Durotak^{®} 280-2287 (51 % solution or solid matter), Durotak^{®} 326-1753 (37% solution or solid matter), Durotak^{®} 280-1753 (33% solution or solid matter), Durotak^{®} 901-1052 (48% solution or solid matter), Durotak^{®} 80-1196 (solid matter), and Durotak^{®} 387-2825 (50% solution).

The adhesive is contained in the plaster of the present invention in an amount of between 40% to 95% by weight of the plaster, preferably between 60 to 90%, more preferably between 70% to 90%, and most preferably between 80% to 90% by weight of the plaster.

The present invention discloses a combination therapy wherein the plaster is used in combination with a systemic treatment of onychomycosis or other systemic treatments for dysfunctions or disorders of nails or nail growth.

Said combination therapy is especially useful for prophylaxis and/or treatment of onychomycosis, onychocryptosis, nail psoriasis, melanonychia striata, white line disease, eczema, chronic onychia,discolored nails, thickened nails, and onychodystrophy.

As used herein, the term "plaster' refers to any device which can be applied to the nail and which comprises a contact layer which is pressed against the nail surface. Suitable plaster devices include plasters or preformed films based upon rubbers, acrylics, urethanes, silicone materials, polyvinylalkylethers, gels, and impregnated microporous membranes. Said plaster device could also be combined with or incorporated or formed into shape of an artificial or fake nail in order to improve cosmetic appearance.

Furthermore, the present invention describes the use of the plaster for prophylaxis and/or treatment of a dysfunction or disorder of nails by adhesively securing to the nail and optionally the surrounding skin of said nail the plaster comprising a layer being designed to be in close contact with the nail and optionally with the surrounding skin. Said layer consists of:
a) an adhesive;
b) at least one skin and/or nail permeation enhancer; and
c) at least one additive selected from the group comprising binders, cross linkers, softeners, solvents, fillers, and/or antioxidants.

Instead of the above-mentioned plaster the embodiment comprising two or more layer can also be used in order prevent and/or a dysfunction or disorder of nail growth by adhesively securing to the nail and optionally the surrounding skin of said nail the plaster consisting of:
a) a backing layer; and
b) a contact layer attached to said backing layer and being designed to be in close contact with the nail and optionally with the surrounding skin;
   said contact layer comprising:
   aa) an adhesive;
   bb) at least one skin and/or nail permeation enhancer; and
   cc) at least one additive selected from the group comprising binders, cross linkers, softeners, solvents, fillers, and/or antioxidants.

Especially, the inventive plaster is highly useful for the prevention and/or treatment of onychomycosis, onychocryptosis, nail psoriasis, melanonychia striata, white line disease, eczema, chronic onychia, discolored nails, thickened nails, and onychodystrophy.

Most likely, said dysfunctions or disorders of nails is induced or caused by fungal, yeast, or bacterial infection of the nails and/or the nail beds or by skin diseases, drugs, physical influences, systemic diseases, contact with chemicals, or idiopathic causes.

Combination therapy disclosed herein can be applied by dysfunction or disorder of nails is induced or caused by fungal, yeast, or bacterial infection of the nails and/or the nail beds, or in the context of skin diseases, such as neurodermitis (atopic eczema), psoriasis and the like, or caused by drugs such as antibiotics, anticoagulative agents, ACE inhibitors, betablockers, thiazides, cytostatic agents, or caused by systemic diseases such as avitaminoses, kidney failure, and heart failure, or caused by chemical compounds such as acids, bases, oxidants and the like, or caused by physical influences resulting in mechanical destruction of the nail plate.

One important aspect of the present invention is that the use of the plasters does not require the procedure of drilling at least one hole into the nail and/or daily scraping of the nail. Another advantage of the present invention is that the plasters are easy to use, convenient and user-friendly.

It is stressed again that the inventive plasters do not contain an antifungal agent. Examples of antifungal agents commonly used in pharmaceutical preparations for treating dysfunctions and disorders (such as mentioned above) of nails can be selected from sertaconazole, fluconazole, butoconazole, chlormidazole, enilconazole, fenticonazole, sulconazole, naftifidine, clioquinol, iodoquinol, rimoprogin, griseofulvin, terbinafine, clotrimazole, itraconazole, tioconazole, miconazole, miconazole nitrate, glyceryl triacetate, tolnaftate, pyrogallol, econazole, isoconazole, terconazole, oxiconazole, voriconazole, amphotericin B, nystatin, tolciclate, sulbentine, haloprogin, ketoconazole, ciclopirox, amorolfine, bifonazole, bifonazole/urea, butenafine/urea, urea, sodium propionate, sodium pyrithione, salicylic acid, and the like.

Furthermore, the inventive plaster can be used in combination with a systemic treatment of a dysfunction or disorder of nails, such as onychomycosis, onychocryptosis, nail psoriasis, melanonychia striata, white line disease, eczema, chronic onychia, discolored nails, thickened nails, and onychodystrophy.

Melanonychia striata or longitudinal melanonychia refers to any linear tan, brown, or black pigmentation within the nail plate that results from increased melanin deposition. Onychomycosis is a fungal infection of the nail caused by dermatophytes, yeasts, or non-dermatophyte moulds. It is the most common nail disorder.

The present invention discloses a method for the prophylaxis and/or treatment of an affected nail, nail bed and surrounding tissue by adhesively securing to the nail and optionally the surrounding skin the plaster, in order to treat a dysfunction or disorder of growth of said nail. Especially, said finger- and/or toenails are affected by onychomycosis, onychocryptosis, nail psoriasis, melanonychia striata, white line disease, eczema, chronic onychia, discolored nails, thickened nails, and onychodystrophy.

One advantage of said inventive use is exhibited by the fact that it does not require drilling holes into the nails and/or daily scraping of the nails.

The inventive use can also be applied in combination with a systemic treatment of a dysfunction or disorder of nails. Especially, a combination of the inventive method with a systemic treatment has been proven effective for the dysfunctions or disorders of nails comprising onychomycosis, onychocryptosis, nail psoriasis, melanonychia striata, white line disease, eczema, chronic onychia, discolored nails, thickened nails, and onychodystrophy.

### Examples

The following examples shall exemplify the present invention.

The plasters or nail patches may preferably comprise a backing layer and/or a release liner. The backing layer, if present, is preferably made from PVC such as Guttagena PVC NBR foil and the release liner is preferably made of PET such as PET foil with both sides siliconized (100 µm).

The following tables present basic formulations for the plaster.

### Example 1:

### Plaster 1: Compounds of the contacting layer for 1.0 cm² plaster

| No. | Compound | Concentration |
|---|---|---|
| 1 | EUDRAGIT^{®} E 100 | 42.2 g |
| 2 | dibutylsebacate | 19.0 g |
| 3 | succinic acid | 3.8 g |
| 4 | acetone | 21.0 g |
| 5 | isopropanol | 2.3 g |
| 6 | ethanol | 11.7 g |

### Equipment:

The solution is prepared in a high-speed stirred tank. The stirrer may be a dissolver disc, for example, which guarantees thorough mixing also at rising viscosity. On a laboratory scale, coating and drying are performed in a laboratory coating unit with integrated dryer (LTSV/LTF by W. Mathis AG, Switzerland).

### Instructions for processing:

Acetone, isopropanol, and ethanol is placed in a stirred tank and EUDRAGIT^{®} E 100 is added in portions over a period of 90 minutes. The stirrer is set to a speed which excludes sediment formation while dissolving EUDRAGIT^{®} E 100. Dibutylsebacate is added swiftly and stirring is continued for another 20 minutes. Thereafter, succinic acid is given to the polymer solution in portions with intensive stirring. After complete addition of succinic acid the polymer solution is stirred for additional 20 minutes.

Coating is performed with the final polymer solution at the following parameters:
- Coating:: approximately 100 g of said polymer solution is applied to the backing layer foil (15 µm thickness, Revatrans ^{®} MN, Tricon GmbH Freiburg) by means of a rotary doctor blade at a nip of 200 µm.
- Drying:: Drying is performed at 60°C for 10 minutes, circulating air: 1500 m³/h, exhaust air: 80 m³/h.

### Product properties:

| | |
|---|---|
| Appearance: | yellowish |
| Solid content: | 65% of polymer solution |
| Water content: | approximately 0.3% (Ph. Eur., "Karl Fischer Method") |
| rel. Density: | d²⁰ = 0.96 g/cm³ |
| Viscosity: | 1500 - 3000 mPa * s (Brookfield II / 6 / 20°C) |
| Adhesive strength: | approximately 3.3 N / cm strip width |
| Test conditions: | 180° peeling angle at a pulling speed of 100 mm / minute |
| Residual solvent: | < 0.05% (total in dried layer) |

### Results:

Double, randomized, multicentric clinical trails were conducted on 20 patients suffering from fungal infection of fingernails.

After a treatment period of six month with a subsequent observation period of one month, 64.7% of the patients treated with the inventive plaster showed positive results and a negative result of mycological culture.

"Positive results" are defined as a decrease in severity of the fungal infection. Either no fungal infection could be detected after the treating period or at the end of the subsequent observation period or only a minor or moderate fungal infection after said treatment period or observation period could be detected.

Side effects were characterized as skin scaling of the tissue surrounding the infected nail (below 10%). The plasters 2 - 7 according to examples 2 - 7 give similar results while the plaster according to the formulation of example 1 is most preferred.

The treatment period can last in isolated cases one year or longer. Normally, the treatment period will be one to several months under the condition that the plaster is replaced weekly.

### Example 2:

### Plaster 2: Compounds of the contacting layer for 1.0 cm² plaster

| No. | Compound | Concentration |
|---|---|---|
| 1 | durotak 387-2825 | 8.80 mg |
| 2 | lactic acid | 0.11 mg |
| 3 | aerosil 200 | 0.33 mg |
| 4 | aluminum acetylacetonate | 0.11 mg |

## Claims

1. Use of a layer being designed to be in close contact with the nail and optionally with the surrounding skin; said layer consisting of:
a) an adhesive;
b) at least one skin and/or nail permeation enhancer; and
c) at least one additive selected from the group comprising binders, cross linkers, softeners, solvents, fillers, and/or antioxidants,
for the manufacture of a plaster for treatment of onychomycoses of nails by adhesively securing to the nail and optionally the surrounding skin of said nail the plaster,
wherein no antifungal agentis present in said plaster.

2. Use of
a) a backing layer; and
b) a contact layer attached to said backing layer and being designed to be in close contact with the nail and optionally with the surrounding skin; said contact layer consisting of:
aa) an adhesive;
bb) at least one skin and/or nail permeation enhancer; and
cc) at least one additive selected from the group comprising binders, cross linkers, softeners, solvents, fillers, and/or antioxidants,
for the manufacture of a plaster for treatment of onychomycoses of nails by adhesively securing to the nail and optionally the surrounding skin of said nail the plaster,
wherein no antifungal agent, is present in said plaster.

3. Use according to claim 2 wherein said backing layer is an occlusive backing layer.

4. Use according to any one of claims 1 - 3 wherein said onychomycoses of nails is induced or caused by fungal or yeast infection of the nails and/or the nail beds, or in the context of skin diseases.

5. Use according to any one of claims 1 - 4 wherein the skin and/or nail permeation enhancer is selected from the group comprising fatty acids, fatty acid esters, fatty acid amides, fatty alcohols, 2-(2-ethoxyethoxy)-ethanol, esters of glycerol, glycerol monolaurate, propylene glycol, polyethylene glycols, unsaturated polyglycolized glycerides, saturated polyglycerides, a partial glyceride of ricinoleic acid, α-hydroxy acids, dimethylsulfoxide, decylmethylsulfoxide, pyrrolidones, lactic acid, myristol, isopropyl myristate, dimethylformamide, dimethylacetamide, sodium dodecylsulfate, phospholipides, and proteolytic enzymes.

6. Use according to any one of claims 1 - 5 wherein the skin and/or nail permeation enhancer is contained in the contact layer in an amount of between 0.7% to 6% by weight of the contact layer.

7. Use according to any one of claims 1 - 6 wherein the additives are contained in the contact layer in an amount of between 15% to 20% by weight of the contact layer.

8. Use according to any one of claims 1 - 7 wherein the adhesive is selected from the group comprising acrylic adhesives, rubber adhesives, and silicone adhesives.

9. Use according to any one of claims 1 - 8 wherein the adhesive is contained in the plaster in an amount of between 80% to 90% by weight of the contact layer.

10. Use according to any one of claims 1 - 9 wherein said use does not require drilling a hole into the nails and/or daily scraping of the nail.

## Patentansprüche

1. Verwendung einer Schicht, die für den engen Kontakt eines Nagels und optional mit der umliegenden Haut gestaltet wurde, wobei die Schicht besteht aus:
a) einem Klebstoff;
b) mindestens einem Haut- und/oder Nagel-Permeationsverstärker; und
c) mindestens einem Zusatzstoff, der aus der Gruppe ausgewählt wird, die Bindemittel, Vernetzungsmittel, Weichmacher, Lösungsmittel, Füllstoffe und/oder Antioxidantien umfasst,
zur Herstellung eines Pflasters zur Behandlung von Onychomykosen des Nagels durch anhaftendes Befestigen am Nagel und optional an der umliegenden Haut des Nagels,
wobei kein antifungaler Wirkstoff in dem besagten Pflaster enthalten sind.

2. Verwendung von
a) einer Trägerschicht; und
b) einer Kontaktschicht, die an der besagten Trägerschicht abgebracht ist und für den engen Kontakt mit dem Nagel und optional mit der umliegenden Haut bestimmt ist, bestehend aus:
aa) einem Klebstoff;
bb) mindestens einem Haut- und/oder Nagel-Permeationsverstärker; und
cc) mindestens einem Zusatzstoff, der aus der Gruppe ausgewählt wird, die Bindemittel, Vernetzungsmittel, Weichmacher, Lösungsmittel, Füllstoffe und/oder Antioxidantien umfasst,
zur Herstellung eines Pflasters zur Behandlung von Onychomykosen des Nagels durch eine anhaftende Befestigung am Nagel und optional an der umliegenden Haut des Nagels,
wobei keine antifungalen Wirkstoffe in dem besagten Pflaster enthalten sind.

3. Verwendung gemäß Anspruch 2, wobei die besagte Trägerschicht eine okklusive Trägerschicht ist.

4. Verwendung gemäß einem der Ansprüche 1 - 3, wobei die besagten Onychomykosen des Nagels durch eine Pilz- oder Hefeinfektion der Nägel und/oder der Nagelbetten herbeigeführt oder verursacht wurde, oder im Zusammenhang mit Hautkrankheiten.

5. Verwendung gemäß einem der Ansprüche 1 - 4, wobei der Haut- und/oder Nagel-Permeationsverstärker aus der Gruppe ausgewählt wird, die Fettsäuren, Fettsäureester, Fettsäureamide, Fettalkohole, 2-(2-Ethoxyethoxy)-ethanol, Glycerolester, Glycerolmonolaureat, Propylenglykol, Polyethylenglykolen, ungesättigten polyglykolisierte Glyceride, gesättigte Polyglyceride, ein Partialglycerid der Ricinolsäure, α-Hydroxysäuren, Dimethylsulfoxid, Decylmethylsulfoxid, Pyrrolidone, Milchsäure, Myristol, Isopropylmyristat, Dimethylformamid, Dimethylacetamid, Natriumdodecylsulfat, Phospholipide und proteolytische Enzymen umfasst.

6. Verwendung gemäß einem der Ansprüche 1 - 5, wobei der Haut- und/oder Nagel-Permeationsverstärker in der Kontaktschicht in einer Menge von 0,7 bis 6 Gewichtsprozent der Kontaktschicht enthalten ist.

7. Verwendung gemäß einem der Ansprüche 1 - 6, wobei die Zusatzstoffe in der Kontaktschicht in einer Menge von 15 bis 20 Gewichtsprozent der Kontaktschicht enthalten sind.

8. Verwendung gemäß einem der Ansprüche 1 - 7, wobei der Klebstoff aus der Gruppe ausgewählt wird, die Acrylkleber, Kautschukkleber und Silikonkleber umfasst.

9. Verwendung gemäß einem der Ansprüche 1 - 8, wobei der Klebstoff in dem Pflaster in einer Menge von 80 bis 90 Gewichtsprozent der Kontaktschicht enthalten ist.

10. Verwendung gemäß einem der Ansprüche 1 - 9, wobei die besagte Verwendung kein Bohren eines Lochs in die Nägel und/oder tägliches Abschaben des Nagels erfordert.

## Revendications

1. Utilisation d'un revêtement dessiné pour le contact étroit avec un ongle et facultativement avec la peau entourant, le revêtement consistant de:
a) un adhésif;
b) au moins un exhausteur de perméation de la peau et/ou de l'ongle; et
c) au moins un additif choisi parmi le groupe comprenant des liants, agents de réticulation, plastifiants, solvants, charges et/ou antioxydants,
pour la production d'un emplâtre pour le traitement d'onychomycoses de l'ongle par l'attache adhésive à l'ongle et facultativement à la peau entourant l'ongle,
où aucun agent antifongique est contenu dans ledit emplâtre.

2. Utilisation de
a) une couche d'appui; et
b) une couche de contact, attachée à ladite couche d'appui et dessinée pour le contact étroit avec un ongle et facultativement avec la peau entourant, cette couche de contact consistant de:
aa) un adhésif;
bb) au moins un exhausteur de perméation de la peau et/ou de l'ongle; et
cc) au moins un additif choisi parmi le groupe comprenant des liants, agents de réticulation, plastifiants, solvants, charges et/ou antioxydants,
pour la production d'un emplâtre pour le traitement d'onychomycoses de l'ongle par l'attache adhésive à l'ongle et facultativement à la peau entourant l'ongle,
où aucun agent antifongique est contenu dans ledit emplâtre.

3. Utilisation selon la revendication 2, où ladite couche d'appui est une couche d'appui occlusive.

4. Utilisation selon une des revendications 1 - 3, où ladite onychomycose de l'ongle est provoquée ou causée d'une infection fongique ou à levure des ongles et/ou des lits de l'ongle, ou dans le contexte de maladies de la peau.

5. Utilisation selon une des revendications 1 - 4, où ledit exhausteur de perméation de la peau et/ou de l'ongle est choisi parmi le groupe comprenant les acides gras, esters d'acides gras, amides d'acides gras, alcools gras, 2-(2-éthoxyéthoxy)-éthanol, esters de glycérol, glycérol monolaurate, propylène glycol, polyéthylèneglycols, glycérides polyglycolisés insaturés, polyglycérides saturés, un glycéride partiel d'acide ricinoléique, acides hydroxy d'alpha, diméthylsulfoxyde, décylméthylsulfoxyde, pyrrolidones, acide lactique, myristol, myristate isopropylique, diméthylformamide, diméthylacétamide, dodécylsulfate de sodium, phospholipides et enzymes protéolytiques.

6. Utilisation selon une des revendications 1 - 5, où l'exhausteur de perméation de la peau et/ou de l'ongle est contenu dans la couche de contact dans une quantité de 0,7 à 6 pourcent en poids de la couche de contact.

7. Utilisation selon une des revendications 1 - 6, où les additifs sont contenus dans la couche de contact dans une quantité de 15 à 20 pourcent en poids de la couche de contact.

8. Utilisation selon une des revendications 1 - 7, où l'adhésif est parmi le groupe comprenant les adhésifs acryliques, les adhésifs caoutchouc et les adhésifs de silicone.

9. Utilisation selon une des revendications 1 - 8, où l'adhésif est contenu dans l'emplâtre dans une quantité de 80 à 90 pourcent en poids de la couche de contact.

10. Utilisation selon une des revendications 1 - 9, où ladite utilisation ne requiert pas de percer un orifice dans les ongles et/ou d'abraser l'ongle chaque jour.
